# EUROPEAN PATENT SPECIFICATION

(11) **EP 1 461 032 B1**
(45) Date of publication and mention of the grant of the patent: **16.07.2008**
(21) Application number: 02801980.0
(22) Date of filing: 20.09.2002
(51) Int. Cl.: A61K 31/506, A61P 17/14

(54) **USE OF C-KIT INHIBITORS FOR PROMOTING HAIR GROWTH**
DIE VERWENDUNG VON C-KITHEMMERN ZUR FÖRDERUNG DES HAARWUCHSES
UTILISATION D'INHIBITEURS DE LA C-KIT POUR STIMULER LA POUSSE DES CHEVEUX

(30) Priority: 20.09.2001 US 323314 P
(43) Date of publication of application: 29.09.2004
(73) Proprietor: AB Science, 75008 Paris (FR)
(72) Inventor: MOUSSY, Alain, F-75006 Paris (FR); KINET, Jean-Pierre, Lexington, MA 02421 (US)
(74) Representative: Warcoin, Jacques
(86) International application number: PCT/IB2002/004290
(87) International publication number: WO 2003/035050

(56) References cited:
- WO-A-01/47507
- WO-A-01/64200
- HEINRICH MICHAEL C ET AL: "Inhibition of c-kit receptor tyrosine kinase activity by STI 571, a selective tyrosine kinase inhibitor" BLOOD, vol. 96, no. 3, 1 August 2000 (2000-08-01), pages 925-932, XP001097629 ISSN: 0006-4971
- XIAOWEI XU ET AL: 'Scarring alopecia associated with mastocytosis' JOURNAL OF CUTANEOUS PATHOLOGY vol. 30, no. 9, 2003, pages 561 - 565
- TANII T. ET AL: 'Increase of mast cells in the alopecia lesion of mice' ACTA DERMATO-VENEREOLOGICA vol. 85, no. 1, 1985, pages 64 - 66
- MAURER M. ET AL: 'Mast cells as modulators of hair follicle cycling' EXPERIMENTAL DERMATOLOGY vol. 4, no. 4, 1995, pages 266 - 271
- MAURER M. ET AL: 'Activated skin mast cells are involved in murine hair follicle regression (catagen).' LABORATORY INVESTIGATION vol. 77, no. 4, 1997, pages 319 - 332
- LANDI G. ET AL: 'Treatment of alopecia areata by mast cell depletion' MINERVA DERMATOLOGICA vol. 39, 1964, pages 102 - 104
- PAUS R. ET AL: 'Mast cell involvement in murine hair growth' DEVELOPMENTALBIOLOGY vol. 163, 1994, pages 230 - 240
- PETERS E.M. ET AL: 'Stress exposure modulates peptidergic innervation and degranulates mast cells in murine skin' BRAIN BEHAV. IMMUN. vol. 19, no. 3, 2005, pages 252 - 262
- ARCK P.C. ET AL: 'Mast cell deficient and neurokinin-1 receptor knockout mice are protected from stress-induced hair growth inhibition' J. MOL. MED. vol. 83, no. 5, 2005, pages 386 - 396
- KULKA M. ET AL: 'Neuropeptides activate human mast cell degranulation and chemokine production' IMMUNOLOGY 2007,
- KALISH R.S. ET AL: 'Alopecia areata: auto-immunity--the evidence is compelling' J. INVESTIG. DERMATOL. SYMP. PROC. vol. 8, no. 2, 2003, pages 164 - 167
- GILHAR A. ET AL: 'Lymphocytes, neuropeptides, and genes involved in alopecia areata' JOURNAL OF CLINICAL INVESTIGATION vol. 117, no. 8, 2007, pages 2019 - 2027
- VILLA I. ET AL: 'Capacity of mouse mast cells to prime T cells and to induce specific antibody responses in vivo' IMMUNOLOGY vol. 102, 2001, pages 165 - 172
- PANIAGUA R.T. ET AL: 'Selective tyrosine kinase inhibition by imatinib mesylate for the treatment of autoimmune arthritis.' JOURNAL OF CLINICAL INVESTIGATION vol. 116, no. 10, pages 2633 - 2642

### SEQUENCE LISTING

<110> AB Science
<120> Use of tyrosine kinase inhibitors for promoting hair growth
<130> D19830 NT
<150> US, 60/323,314
   <151> 2001-09-20
<160> 5
<170> PatentIn Ver. 2.1
<210> 1
   <211> 976
   <212> PRT
   <213> Homo sapiens
<220>
   <223> Human c-kit
<400> 1
<210> 2
   <211> 30
   <212> DNA
   <213> Homo sapiens
<220>
   <223> Primer
<400> 2
   aagaagagat ggtacctcga ggggtgaccc 30
<210> 3
   <211> 33
   <212> DNA
   <213> Homo sapiens
<220>
   <223> Primer
<400> 3
   ctgcttcgcg gccgcgttaa ctcttctcaa cca 33
<210> 4
   <211> 20
   <212> DNA
   <213> Homo sapiens
<220>
   <223> Primer
<400> 4
   agctcgttta gtgaaccgtc 20
<210> 5
   <211> 20
   <212> DNA
   <213> Homo sapiens
<220>
   <223> Primer
   <400> 5
   gtcagacaaa atgatgcaac 20

## Description

The present invention relates to the use of a c-kit inhibitor selected from the group consisting of N-phenyl-2-pyrimidine-amine for preparing a medicament, or in a cosmetic composition, for treating alopecia areata and alopecia resulting from emotional distress.

Hairs are filamentous, keratinized structures consisting of a shaft and a root. The shaft is composed of specialized keratinocytes. The root lies within the hair follicle and comprises the germinative matrix and the keratogenous zone. The germinative matrix consists of pluripotent cells showing mitotic activity giving rise to the hair and its surrounding inner root sheath. Cells arising mitotically from this group move apically and differentiate along several different routes.

Hair growth depends on proliferation of hair follicle matrix cells. It alternates between phases of activity and rest. The anagen phase is a period of growth lasting for two to six years. During this time, the follicle is long and deep, and produces thick, well-pigmented hair. Usually, about 90% of all scalp hairs are in the anagen phase at a given time. This growth phase is followed by the catagen phase for few weeks, which corresponds to the follicle base shrinking. The resting period, or telogen phase, lasts for two to four months. In this phase, the follicle withers even further. Following the telogen phase, the next anagen phase begins, and the old hair is dislodged and falls out to make room for a new hair.

Hair loss (alopecia areata and androgenetic alopecia) is extremely common among healthy adult humans, especially men. Indeed, hair loss affects approximately 50% of men at some point in their lives.

Alopecia androgenetic is an inherited condition, caused by a genetically determined sensitivity to the effects of dihydrotestosterone, which is believed to shorten the anagen phase of the hair cycle, causing miniaturisation of the follicles, and producing progressively finer hairs. Alopecia can also be induced by chemical agents or physical agents (e.g., during anti-cancer chemotherapy), and the condition also results from specific disease states and factors (emotional distress) and with increasing age. Alopecia typically is attributable to a disturbance in the hair renewal cycle leading to acceleration of the frequency of the cycles, which results in a shift in the population of follicles from the anagen phase to telogen. Ultimately, the hair follicles degenerate and a decrease in the number hairs in the affected area of the scalp or skin is observed.

In addition, hair loss can have a serious psychological impact. Studies have shown that hair loss can be associated with low self esteem, depression, introversion, and feelings of unattractiveness. This is reinforced by attitudes in Western society, which place great value on youthful appearance and attractiveness. Some studies have shown that based on appearance alone, men with hair loss are seen as less attractive, less assertive, less likeable, and less successful than men without hair loss.

As a result, there is real need in the development of cosmetic and clinical treatments for promoting hair growth, preventing or minimizing hair loss.

As of today no treatment has been shown to be effective. Mud preparations and plant extracts have been proposed for enhancing hair growth in US 5,798,341, US 5,767,152, US 5,753,713, US 5,750,107, US 5,741,816, US 5,739,111, US 5,723,149, US 5,679,378, US 5,674,497, US 5,663,160, US 5,656,300, US 5,643,898, and US 4,139,619.

2,4-diamino-6-piperidinopyrimidine 3-oxide (minoxidil) and its derivatives have been described in US 4,596,812, EP 522,964, EP 420,707, EP 459,890 and EP 519,819 for slowing hair loss. However, topical application of minoxidil and other agents is only partially effective and suffers from a number of serious side effects in many patients.

Other methods for treating alopecia include :
- the administration of anol, anethole and analogs with various mixture of herbs such as umbelliferae, magnoliaceae, labiatae and rutaceae (US 5,422,100),
- topical application of retinoic acid to the skin and hair (US 5,514,672),
- treatment of hair and scalp which chelating agents, gellan gum, vitamin precursors and derivatives, biotin, γ-linolenic acid, menthol, liposomes, various conditioners, humectants, and folic acid (US 5,523,078),
- cosmetic preparations containing solid particles of gold, silver or platinum (US 5,587,168).
- topical compositions comprising an aryl-substituted ethylene for inducing, maintaining or increasing hair growth (EP 0403238),
- and compositions containing others compounds such as estrogens, sulfur, sulfide ions, vasodilators, inorganic selenium compounds, amino acids and protein extracts, garlic powder, brewer's yeast, grapefruit juice, acetic acid and kelp (US 5,629,002 and US 5,674,510).

Despite the above mentioned numerous approaches, effective solutions for stimulating hair growth and for preventing or minimizing hair loss continue to remain elusive.

Numerous factors affect the hair growth cycle including heredity, hormonal deficiencies or imbalances, diet, stress, chemotherapy or aging. For example, it has been shown that various growth factors, steroid hormones, dermo-epithelial interaction, dermal vascularity, neuroectodermal factors, and the immune system are implicated; Stenn et al., Dermatol. Clin., 14, 167-96 (1996) and Lindler et al., Am. J Pathol., 151, 1601-17 (1997).

More recently, alopecia areata has been considered to be a T-cell mediated autoimmune disease of the hair follicle Freyschmidt-Paul P et al, Curr Pharm Des 2001 Feb;7(3):213-30. In accordance with this mechanism, possible future therapeutic approaches include application of immunosuppressive cytokines like TGF-β and IL-10.

Ruckert R. et al, Br J Dermatol 2000 Nov;143(5):1036-9 also observed that hair loss following skin inflammation may in part be mediated by keratinocyte (KC) apoptosis. The effects of different cytokines or other apoptosis stimulating agents such as interferon IFN-γ or TNF-α on KC apoptosis have been demonstrated in vitro and in vivo.

Furthermore, histological investigations have shown that in C57BL mice and +/+ mice, the number of dermal mast cells in the bald areas was greater than that in controls.

Translation of these data to the human condition is rendered difficult by the complexity of the mechanisms involved in the "classical male pattern alopecia". Nevertheless, it has been found that the number of inflammatory infiltrates around the follicular infundibula of the alopecic vertices and non-alopecic occiputs of male pattern alopecia patients are significantly greater than the corresponding control value. Of interest, the number of mast cells in the widened fibrous tracts in the vertices of male pattern alopecia patients was found significantly greater than those in the adventitial fibrotic sheaths of control subjects and the non-alopecic occiputs of male pattern alopecia patients.

Mast cells (MC) are tissue elements derived from a particular subset of hematopoietic stem cells that express CD34, c-kit and CD 13 antigens (Kirshenbaum et al, Blood. 94: 2333-2342, 1999 and Ishizaka et al, Curr Opin Immunol. 5: 937-43, 1993). Immature MC progenitors circulate in the bloodstream and differentiate in tissues. These differentiation and proliferation processes are under the influence of cytokines, one of utmost importance being Stem Cell Factor (SCF), also termed Kit ligand (KL), Steel factor (SL) or Mast Cell Growth Factor (MCGF). SCF receptor is encoded by the protooncogene c-kit, that belongs to type III receptor tyrosine kinase subfamily (Boissan and Arock, J Leukoc Biol. 67: 135-48, 2000). This receptor is also expressed on others hematopoietic or non hematopoietic cells. Ligation of c-kit receptor by SCF induces its dimerization followed by its transphosphorylation, leading to the recruitement and activation of various intracytoplasmic substrates. These activated substrates induce multiple intracellular signaling pathways responsible for cell proliferation and activation (Boissan and Arock, 2000). Mast cells are characterized by their heterogeneity, not only regarding tissue location and structure but also at the functional and histochemical levels (Aldenborg and Enerback., Histochem. J. 26: 587-96, 1994 ; Bradding et al. J Immunol. 155: 297-307, 1995 ; Irani et al, J Immunol. 147: 247-53, 1991 ; Miller et al, Curr Opin Immunol. 1: 637-42, 1989 and Welle et al, J Leukoc Biol. 61: 233-45, 1997).

In connection with the invention, it is proposed that mast cells play a crucial role in alopecia, in that they produce a large variety of mediators categorized here into three groups:
- preformed granule-associated mediators (histamine, proteoglycans, and neutral proteases),
- lipid-derived mediators (prostaglandins, thromboxanes and leucotrienes),
- and various cytokines (IL-1, IL-2, IL-3, IL-4, IL-5, IL-6, IL-8, TNF-α, GM-CSF, MIP-1a, MIP-1b and IFN-γ).

Then, liberation by activated mast cells of mediators (TNF- α, leucotrienes, prostaglandines etc...) can induce inflammation around follicles leading to apoptosis of cells in the germinative matrix. This support that the inflammatory process mediated by mast cells is, at least in part, responsible for the development of alopecia. Local therapeutic strategies aiming at blocking the activation and the survival of mast cells, for instance through inhibition of c-kit or c-kit signaling can thus be beneficial and could help to decrease hair-loss in such condition.

More specifically, the present invention proposes to use c-kit specific kinase inhibitors to inhibit mast cell proliferation, survival and activation. A new route for promoting hair growth, preventing or minimizing hair loss is provided, which consists of destroying mast cells playing a role in the apoptosis of cells in the hair follicles. It has been found that tyrosine kinase inhibitors and more particularly c-kit inhibitors are especially suited to reach this goal.

### Description

The present invention relates to the use of a c-kit inhibitor selected from the group consisting of N-phenyl-2-pyrimidine-amine of formula II:

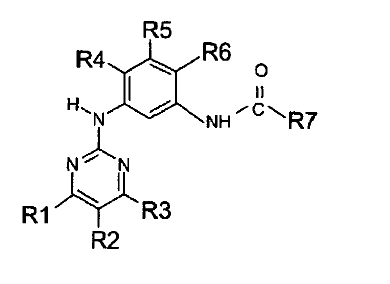

Wherein R1, R2 and R3 are independently chosen from H, F, Cl, Br, I, a C1-C5 alkyl or a cyclic or heterocyclic group, especially a pyridyl group;
R4, R5 and R6 are independently chosen from H, F, Cl, Br, I, a C1-C5 alkyl, especially a methyl group;
and R7 is a phenyl group bearing at least one substituent, which in turn possesses at least one amino group,
for preparing a medicament for treating alopecia areata and alopecia resulting from emotional distress.

Preferably, R7 is

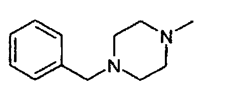

For example, said inhibitor is the 4-(4-methylpiperazine-1-ylmethyl)-N-[4-methyl-3-(4-pyridine-3-yl)pyrimidine-2 ylamino)phenyl]-benzamide corresponding to the following formula :

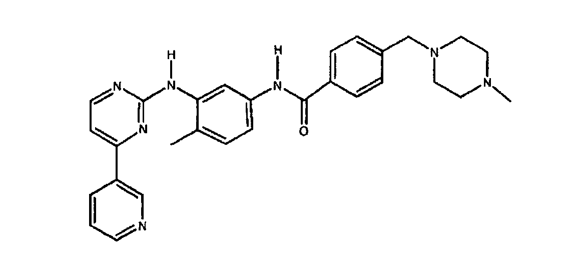

The preparation of this compound is described in example 21 of EP 564 409 and the β-form, which is particularly useful is described in WO 99/03854.

The invention is also aimed at the cosmetic use of a c-kit inhibitor selected from the group consisting of N-phenyl-2-pyrimidine-amine of formula II:

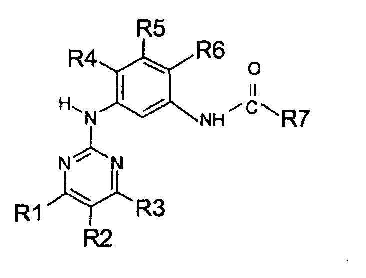

Wherein R1, R2 and R3 are independently chosen from H, F, Cl, Br, I, a C1-C5 alkyl or a cyclic or heterocyclic group, especially a pyridyl group;
R4, R5 and R6 are independently chosen from H, F, Cl, Br, I, a C1-C5 alkyl, especially a methyl group;
and R7 is a phenyl group bearing at least one substituent, which in turn possesses at least one amino group,
for treating alopecia areata and alopecia resulting from emotional distress.

Preferably R7 is 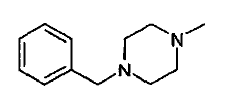 For example, said inhibitor is the 4-(4-methylpiperazine-1-ylmethyl)-N-[4-methyl-3-(4-pyridine-3-yl)pyrimidine-2 ylamino)phenyl]-benzamide.

The invention also concerns a dermatology or cosmetic composition for topical administration which is in the form of a gel, paste, ointment, cream, lotion, liquid suspension aqueous, aqueous-alcoholic or, oily solutions, or dispersions of the lotion or serum type, or anhydrous or lipophilic gels, or emulsions of liquid or semi-solid consistency of the milk type, obtained by dispersing a fatty phase in an aqueous phase or vice versa, or of suspensions or emulsions of soft, semi-solid consistency of the cream or gel type, or alternatively of microemulsions, of microcapsules, of microparticles or of vesicular dispersions to the ionic and/or nonionic type and comprising a c-kit inhibitor as defined above.

It optionally comprises at least one compound selected from the group consisting of :
- 2,4-diamino-6-piperidinopyrimidine 3-oxide (minoxidil) and its derivatives,
- anol, anethole and analogs with various mixture of plant extracts such as umbelliferae, magnoliaceae, labiatae and rutaceae,
- retinoic acid, chelating agents, gellan gum, vitamin precursors and derivatives, biotin, γ-linolenic acid, menthol, liposomes, various conditioners, humectants, folic acid, particles of gold, silver or platinum (US 5,587,168), estrogens, sulfur, sulfide ions, vasodilators, inorganic selenium compounds, amino acids and protein extracts.

Pharmaceutical compositions suitable for use in the invention include compositions wherein c-kit inhibitors are contained in an effective amount to achieve the intended purpose. The determination of an effective dose is well within the capability of those skilled in the art. A therapeutically effective dose refers to that amount of active ingredient, which ameliorates the symptoms or condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio of toxic to therapeutic effects is the therapeutic index, and it can be expressed as the ratio, LD50/ED50. Pharmaceutical compositions which exhibit large therapeutic indices are preferred. As mentioned above, a c-kit inhibitor according to the invention is unable to promote death of IL-3 dependent cells cultured in presence of IL-3.

The composition according to the invention comprises any ingredient commonly used in dermatology and cosmetic. It may comprise at least one ingredient selected from hydrophilic or lipophilic gelling agents, hydrophilic or lipophilic active agents, preservatives, emollients, viscosity enhancing polymers, humectants, surfactants, preservatives, antioxidants, solvents, and fillers, antioxidants, solvents, perfumes, fillers, screening agents, bactericides, odor absorbers and coloring matter.

As oils which can be used in the invention, mineral oils (liquid paraffin), vegetable oils (liquid fraction of shea butter, sunflower oil), animal oils, synthetic oils, silicone oils (cyclomethicone) and fluorinated oils may be mentioned. Fatty alcohols, fatty acids (stearic acid) and waxes (paraffin, carnauba, beeswax) may also be used as fatty substances.

As emulsifiers which can be used in the invention, glycerol stearate, polysorbate 60 and the PEG-6/PEG-32/glycol stearate mixture are contemplated.
As hydrophilic gelling agents, carboxyvinyl polymers (carbomer), acrylic copolymers such as acrylate/alkylacrylate copolymers, polyacrylamides, polysaccharides such as hydroxypropylcellulose, clays and natural gums may be mentioned, and as lipophilic gelling agents, modified clays such as bentones, metal salts of fatty acids such as aluminum stearates and hydrophobic silica, or alternatively ethylcellulose and polyethylene may be mentioned.

As hydrophilic active agents, proteins or protein hydrolysates, amino acids, polyols, urea, allantoin, sugars and sugar derivatives, vitamins, starch and plant extracts, in particular those of Aloe vera may be used.

As lipophilic active, agents, retinol (vitamin A) and its derivatives, tocopherol (vitamin E) and its derivatives, essential fatty acids, ceramides and essential oils may be used. These agents add extra moisturizing or skin softening features when utilized.

If desired, a known gelling agent may be added to the composition of the invention. Suitable gelling agents include a synthetic high molecular weight crosslinked polymer of acrylic acid, more specifically an acrylate/C.sub.10-30 alkyl acrylate copolymer available for example under the trade name CARBOMER 1342. Other suitable gelling agents include cellulose and cellulose derivatives such as dihydroxyethyl cellulose (tradename ULTRAGEL).

In addition, a surfactant can be included in the composition so as to provide deeper penetration of the ingredients and of the c-kit inhibitor.

Among the contemplated ingredients, the invention embraces penetration enhancing agents selected for example from the group consisting of mineral oil, water, ethanol, triacetin, glycerin and propylene glycol; cohesion agents selected for example from the group consisting of polyisobutylene, polyvinyl acetate and polyvinyl alcohol, and thickening agents.

Chemical methods of enhancing topical absorption of drugs are well known in the art. For example, compounds with penetration enhancing properties include sodium lauryl sulfate (Dugard, P. H. and Sheuplein, R. J., "Effects of Ionic Surfactants on the Permeability of Human Epidermis: An Electrometric Study," J. Ivest. Dermatol., V.60, pp. 263-69, 1973), lauryl amine oxide (Johnson et. al., US 4,411,893), azone (Rajadhyaksha, US 4,405,616 and 3,989,816) and decylmethyl sulfoxide (Sekura, D. L. and Scala, J., "The Percutaneous Absorption of Alkylmethyl Sulfides," Pharmacology of the Skin, Advances In Biolocy of Skin, (Appleton-Century Craft) V. 12, pp. 257-69, 1972). It has been observed that increasing the polarity of the head group in amphoteric molecules increases their penetration-enhancing properties but at the expense of increasing their skin irritating properties (Cooper, E. R. and Berner, B., "Interaction of Surfactants with Epidermal Tissues: Physiochemical Aspects," Surfactant Science Series, V. 16, Reiger, M. M. ed. (Marcel Dekker, Inc.) pp. 195-210, 1987).

Suitable solvents include alkyl esters of fatty acids, preferably C.sub.1-12, more preferably C.sub.3-10, alkyl esters of saturated or unsaturated fatty acids containing 8-22 carbon atoms. Particularly preferred solvents include isopropyl myristate, octyl palmitate, WIKENOL 161 (a mixture of esters), etc. Alcohols such as ethanol, propanol, isopropanol, propylene glycol, etc., as well as aqueous mixtures of these alcohols may also be used.

A second class of chemical enhancers are generally referred to as co-solvents. These materials are absorbed topically relatively easily, and, by a variety of mechanisms, achieve permeation enhancement for some drugs. Ethanol (Gale et. al., U.S. Pat. No. 4,615,699 and Campbell et. al., U.S. Pat. Nos. 4,460,372 and 4,379,454), dimethyl sulfoxide (US 3,740,420 and 3,743,727, and US 4,575,515), and glycerine derivatives (US 4,322,433) are a few examples of compounds which have shown an ability to enhance the absorption of various compounds.

Any formulation which allows delivery of the active compounds of the present invention to the skin, hair and hair follicles.

Therefore, the invention also contemplates a cosmetic composition comprising a c-kit inhibitor as defined above, and at least one ingredient as depicted above suitable for a topical administration. Preferably, the composition is formulated for the delivery of the c-kit inhibitor to the skin, hair or hair follicles, such as a hair-conditioning composition.

## Claims

1. The use of a c-kit inhibitor selected from the group consisting of N-phenyl-2-pyrimidine-amine of formula II: 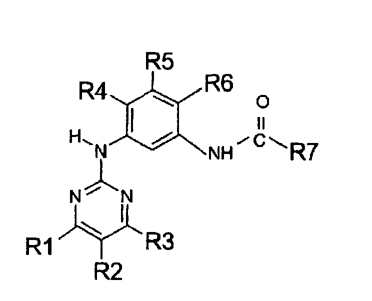 Wherein R1, R2 and R3 are independently chosen from H, F, Cl, Br, I, a C1-C5 alkyl or a cyclic or heterocyclic group, especially a pyridyl group;
R4, R5 and R6 are independently chosen from H, F, Cl, Br, I, a C1-C5 alkyl, especially a methyl group;
and R7 is a phenyl group bearing at least one substituent, which in turn possesses at least one amino group,
for preparing a medicament for treating alopecia areata and alopecia resulting from emotional distress.

2. The use according to claim 1, wherein R7 is

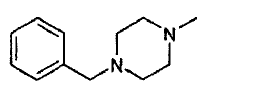

3. The use according to claim 2, wherein said inhibitor is the 4-(4-methylpiperazine-1-ylmethyl)-N-[4-methyl-3-(4-pyridine-3-yl)pyrimidine-2 ylamino)phenyl]-benzamide.

4. The cosmetic use of a c-kit inhibitor selected from the group consisting of N-phenyl-2-pyrimidine-amine of formula II: 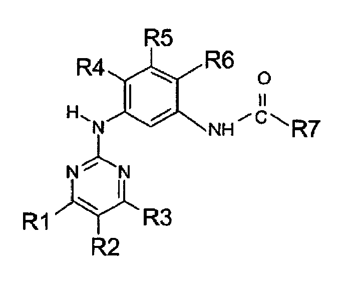 Wherein R1, R2 and R3 are independently chosen from H, F, Cl, Br, I, a C1-C5 alkyl or a cyclic or heterocyclic group, especially a pyridyl group;
R4, R5 and R6 are independently chosen from H, F, Cl, Br, I, a C1-C5 alkyl, especially a methyl group;
and R7 is a phenyl group bearing at least one substituent, which in turn possesses at least one amino group,
for treating alopecia areata and alopecia resulting from emotional distress.

5. The use according to claim 4, wherein R7 is

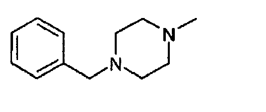

6. The use according to claim 5, wherein said inhibitor is the 4-(4-methylpiperazine-1-ylmethyl)-N-[4-methyl-3-(4-pyridine-3-yl)pyrimidine-2 ylamino)phenyl]-benzamide.

7. A cosmetic composition suitable for topical administration which is in the form of a gel, paste, ointment, cream, lotion, liquid suspension aqueous, aqueous-alcoholic or, oily solutions, or dispersions of the lotion or serum type, or anhydrous or lipophilic gels, or emulsions of liquid or semi-solid consistency of the milk type, obtained by dispersing a fatty phase in an aqueous phase or vice versa, or of suspensions or emulsions of soft, semi-solid consistency of the cream or gel type, or alternatively of microemulsions, of microcapsules, of microparticles or of vesicular dispersions to the ionic and/or nonionic type, comprising a c-kit inhibitor as defined in one of claims 1 to 3.

8. A dermatology composition suitable for topical administration which is in the form of a gel, paste, ointment, cream, lotion, liquid suspension aqueous, aqueous-alcoholic or, oily solutions, or dispersions of the lotion or serum type, or anhydrous or lipophilic gels, or emulsions of liquid or semi-solid consistency of the milk type, obtained by dispersing a fatty phase in an aqueous phase or vice versa, or of suspensions or emulsions of soft, semi-solid consistency of the cream or gel type, or alternatively of microemulsions, of microcapsules, of microparticles or of vesicular dispersions to the ionic and/or nonionic type, comprising a c-kit inhibitor as defined in one of claims 1 to 3.

9. A composition according to claim 8 or 9 which comprises at least one ingredient selected from hydrophilic or lipophilic gelling agents, hydrophilic or lipophilic active agents, emollients, viscosity enhancing polymers, humectants, surfactants, preservatives, antioxidants, solvents, and fillers.

10. A composition according to claim 7 or 8, which is formulated for the delivery of the c-kit inhibitor to the skin, hair or hair follicles.

11. A composition according to claim 8, which is a hair-conditioning composition.

12. A composition according to claim 7 or 8 further comprising at least one compound selected from the group consisting of:
- 2,4-diamino-6-piperidinopyrimidine 3-oxide (minoxidil) and its derivatives,
- anol, anethole and analogs with various mixture of plant extracts such as umbelliferae, magnoliaceae, labiatae and rutaceae,
- retinoic acid, chelating agents, gellan gum, vitamin precursors and derivatives, biotin, γ-linolenic acid, menthol, liposomes, various conditioners, humectants, folic acid, particles of gold, silver or platinum, estrogens, sulfur, sulfide ions, vasodilators, inorganic selenium compounds, amino acids and protein extracts.

## Patentansprüche

1. Verwendung eines c-Kit-Hemmers, ausgewählt aus der Gruppe bestehend aus N-Phenyl-2-pyrimidinamin der Formel II: 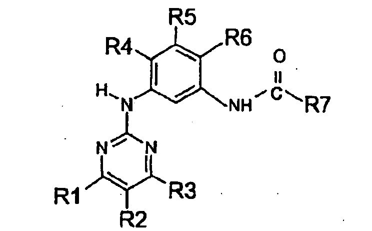 wobei, R1, R2 und R3 unabhängig ausgewählt sind aus H, F, C1, Br, I und einem C1-C5-Alkyl oder einer cyclischen oder heterocyclischen Gruppe, insbesondere einer Pyridylgruppe;
R4, R5 und R6 unabhängig ausgewählt sind aus H, F, Cl, Br, I und einem C1-C5-Alkyl, insbesondere einer Methylgruppe;
und R7 eine Phenylgruppe ist, die wenigstens einen Substituenten aufweist, der wiederum wenigstens eine Aminogruppe besitzt,
zur Herstellung eines Arzneimittels zum Behandeln von Alopecia areata und Alopecia, die von emotionaler Belastung herrührt.

2. Verwendung nach Anspruch 1, wobei R7 ist

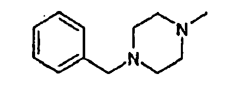

3. Verwendung nach Anspruch 2, wobei der Hemmer das 4-(4-Methylpiperazin-1-ylmethyl)-N-[4-methyl-3-(4-pyridin-3-yl)pyrimidin-2 ylamino)phenyl]-benzamid ist.

4. Kosmetische Verwendung eines c-Kit-Hemmers, ausgewählt aus der Gruppe bestehend aus N-Phenyl-2-pyrimidinamin der Formel II: 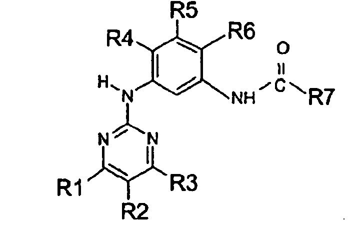 wobei, R1, R2 und R3 unabhängig ausgewählt sind aus H, F, Cl, Br, I und einem C1-C5-Alkyl oder einer cyclischen oder heterocyclischen Gruppe, insbesondere einer Pyridylgruppe;
R4, R5 und R6 unabhängig ausgewählt sind aus H, F, Cl, Br, I und einem C1-C5-Alkyl, insbesondere einer Methylgruppe;
und R7 eine Phenylgruppe ist, die wenigstens einen Substituenten aufweist, der wiederum wenigstens eine Aminogruppe besitzt,
zur Behandlung von Alopecia areata und Alopecia, die von emotionaler Belastung herrührt.

5. Verwendung nach Anspruch 4, wobei R7 ist

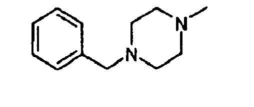

6. Verwendung nach Anspruch 5, wobei der Hemmer das 4-(4-Methylpiperazin-1-ylmethyl)-N-[4-methyl-3-(4-pyridin-3-yl)pyrimidin-2 ylamino)phenyl]-benzamid ist.

7. Kosmetische Zusammensetzung, die zur topischen Anwendung geeignet ist und die Form eines Gels, einer Paste, einer Salbe, einer Creme, einer Lotion, von wässrigen, alkoholisch-wässrigen oder öligen Flüssigsuspensions-Lösungen oder Dispersionen vom Lotions- oder Serumstyp oder wasserfreien oder lipophilen Gelen oder Emulsionen von flüssiger oder halbfester Konsistenz vom Milchtyp, erhalten durch Dispergieren einer Fettphase in einer wässrigen Phase oder umgekehrt, oder von Suspensionen oder Emulsionen von weicher, halbfester Konsistenz vom Creme- oder Geltyp oder alternativ von Mikroemulsionen, von Mikrokapseln, von Mikropartikeln oder von vesikulären Dispersionen zum ionischen und/oder nichtionischen Typ aufweist, umfassend einen c-Kit-Hemmer nach einem der Ansprüche 1 bis 3.

8. Dermatologiezusammensetzung, die zur topischen Anwendung geeignet ist und die Form eines Gels, einer Paste, einer Salbe, einer Creme, einer Lotion, von wässrigen, alkoholisch-wässrigen oder öligen Flüssigsuspensions-Lösungen oder Dispersionen vom Lotions- oder Serumstyp oder wasserfreien oder lipophilen Gelen oder Emulsionen von flüssiger oder halbfester Konsistenz vom Milchtyp, erhalten durch Dispergieren einer Fettphase in einer wässrigen Phase oder umgekehrt, oder von Suspensionen oder Emulsionen von weicher, halbfester Konsistenz vom Creme- oder Geltyp oder alternativ von Mikroemulsionen, von Mikrokapseln, von Mikropartikeln oder von vesikulären Dispersionen zum ionischen und/oder nichtionischen Typ aufweist, umfassend einen c-Kit-Hemmer nach einem der Ansprüche 1 bis 3.

9. Zusammensetzung nach Anspruch 8 oder 9, welche wenigstens einen Bestandteil umfasst, der aus hydrophilen oder lipophilen Geliermitteln, hydrophilen oder lipophilen Wirkstoffen, Erweichungsmitteln, Polymeren zur Erhöhung der Viskosität, Benetzungsmitteln, oberflächenaktiven Stoffen, Konservierungsmitteln, Antioxidationsmitteln, Lösemitteln und Füllmitteln ausgewählt ist.

10. Zusammensetzung nach Anspruch 7 oder 8, welche zur Applikation des c-Kit-Hemmers auf die Haut, das Haar oder Haarfollikel formuliert ist.

11. Zusammensetzung nach Anspruch 8, welche eine Zusammensetzung zur Haarkonditionierung ist.

12. Zusammensetzung nach Anspruch 7 oder 8, ferner umfassend wenigstens eine Verbindung, ausgewählt aus der Gruppe bestehend aus:
- 2,4-Diamino-6-piperidinpyrimidin 3-oxid (Minoxidil) und ihren Derivaten,
- Anol, Anethol und Analogen mit verschiedener Mischung von Pflanzenextrakten, wie beispielsweise Umbelliferae, Magnoliaceae, Labiatae und Rutaceae,
- Retinsäure, Chelatbildnern, Gellangummi, Vitaminvorstufen und Derivaten, Biotin, γ-Linolsäure, Menthol, Liposomen, verschiedenen Zusatzstoffen, Benetzungsmitteln, Folsäure, Partikeln von Gold, Silber oder Platin, Östrogenen, Sulfur, Sulfidionen, Vasodilatatoren, anorganischen Selenverbindungen, Aminosäuren und Proteinextrakten.

## Revendications

1. Utilisation d'un inhibiteur de c-kit choisi dans le groupe constitué par la N-phényl-2-pyrimidine-amine de formule II : 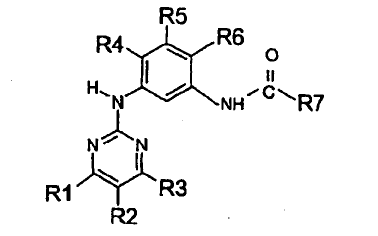 dans laquelle R1, R2 et R3 sont indépendamment choisis parmi H, F, Cl, Br, I, un radical alkyle en C1-C5 ou un groupe cyclique ou hétérocyclique, en particulier un groupe pyridyle ;
R4, R5 et R6 sont indépendamment choisis parmi H, F, Cl, Br, I, un radical alkyle en C1-C5, en particulier un groupe méthyle ;
et R7 est un groupe phényle portant au moins un substituant, qui à son tour possède au moins un groupe amino,
pour préparer un médicament destiné au traitement de l'alopécie areata et de l'alopécie résultant d'une détresse émotionnelle.

2. Utilisation selon la revendication 1, dans laquelle R7 est

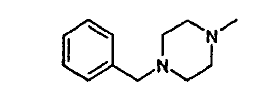

3. Utilisation selon la revendication 2, dans laquelle ledit inhibiteur est le 4-(4-méthylpipérazin-1-ylméthyl)-N-[4-méthyl-3-(4-pyridin-3-yl)pyrimidin-2-ylamino)phényl]-benzamide.

4. Utilisation cosmétique d'un inhibiteur de c-kit choisi dans le groupe constitué par la N-phényl-2-pyrimidine-amine de formule II : 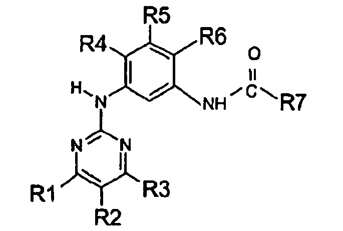 dans laquelle R1, R2 et R3 sont indépendamment choisis parmi H, F, Cl, Br, I, un radical alkyle en Cl-C5 ou un groupe cyclique ou hétérocyclique, en particulier un groupe pyridyle ;
R4, R5 et R6 sont indépendamment choisis parmi H, F, Cl, Br, I, un radical alkyle en C1-C5, en particulier un groupe méthyle ;
et R7 est un groupe phényle portant au moins un substituant, qui à son tour possède au moins un groupe amino,
pour le traitement de l'alopécie areata et de l'alopécie résultant d'une détresse émotionnelle.

5. Utilisation selon la revendication 4, dans laquelle R7 est

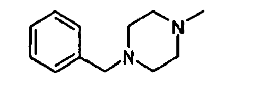

6. Utilisation selon la revendication 5, dans laquelle ledit inhibiteur est le 4-(4-méthylpipérazin-1-ylméthyl)-N-(4-méthyl-3-(4-pyridin-3-yl)pyrimidin-2-ylamino)phényl]-benzamide.

7. Composition cosmétique convenant pour une administration par voie topique, qui est sous la forme d'un gel, d'une pâte, d'une pommade, d'une crème, d'une lotion, d'une suspension liquide, de solutions aqueuses, aqueuses-alcooliques ou huileuses, ou de dispersions du type lotion ou sérum, ou de gels anhydres ou lipophiles, ou d'émulsions ayant une consistance liquide ou semi-solide du type lait, obtenues par dispersion d'une phase grasse dans une phase aqueuse ou vice versa, ou de suspensions ou émulsions ayant une consistance molle, semi-solide, du type crème ou gel, ou en variante de microémulsions, de microcapsules, de microparticules ou de dispersions vésiculaires du type ionique et/ou non-ionique, comprenant un inhibiteur de c-kit tel que défini dans l'une des revendications 1 à 3.

8. Composition dermatologique convenant pour une administration par voie topique, qui est sous la forme d'un gel, d'une pâte, d'une pommade, d'une crème, d'une lotion, d'une suspension liquide, de solutions aqueuses, aqueuses-alcooliques ou huileuses, ou de dispersions du type lotion ou sérum, ou de gels anhydres ou lipophiles, ou d'émulsions ayant une consistance liquide ou semi-solide du type lait, obtenues par dispersion d'une phase grasse dans une phase aqueuse ou vice versa, ou de suspensions ou émulsions ayant une consistance molle, semi-solide, du type crème ou gel, ou en variante de microémulsions, de microcapsules, de microparticules ou de dispersions vésiculaires du type ionique et/ou non-ionique, comprenant un inhibiteur de c-kit tel que défini dans l'une des revendications 1 à 3.

9. Composition selon la revendication 8 ou 9, qui comprend au moins un ingrédient choisi parmi les agents gélifiants hydrophiles ou lipophiles, les agents actifs hydrophiles ou lipophiles, les émollients, les polymères amplifiant la viscosité, les humectants, les tensioactifs, les conservateurs, les antioxydants, les solvants, et les charges.

10. Composition selon la revendication 7 ou 8, qui est formulée pour la délivrance de l'inhibiteur de c-kit à la peau, aux cheveux ou aux follicules capillaires.

11. Composition selon la revendication 8, qui est une composition de conditionnement des cheveux.

12. Composition selon la revendication 7 ou 8, comprenant en outre au moins un composé choisi dans le groupe constitué par :
- le 3-oxyde de 2,4-diamino-6-pipéridinopyrimidine (minoxidil) et ses dérivés,
- l'anol, l'anéthol et analogues avec divers mélanges d'extraits végétaux tels qu'umbelliferae, magnoliaceae, labiatae et rutaceae,
- l'acide rétinoïque, les agents chélatants, la gomme gellane, les précurseurs et dérivés de vitamines, la biotine, l'acide γ-linolénique, le menthol, les liposomes, divers conditionneurs, les humectants, l'acide folique, les particules d'or, d'argent ou de platine, les oestrogènes, le soufre, les ions sulfure, les vasodilatateurs, les composés inorganiques du sélénium, les acides aminés et les extraits protéiques.
